(19) Europäisches Patentamt

European Patent Office

Office européen des brevets

(11) **EP 1 249 227 B1**

(12) **FASCICULE DE BREVET EUROPEEN**

(45) Date de publication et mention de la délivrance du brevet:
**04.08.2004 Bulletin 2004/32**

(51) Int Cl.7: **A61K 7/48**

(21) Numéro de dépôt: **02290860.2**

(22) Date de dépôt: **05.04.2002**

(54) **Composition anti-rides a effet immédiat a base de dispersion aqueuse d'au moins une charge minérale.**

Zusammensetzung gegen Falten mit direktem Effekt, basierend auf einer wässrigen Dispersion mindestens eines anorganischen Füllstoffes

Antiwrinkle composition with immediate effect based on an aqueous dispersion of at least one mineral filler

(84) Etats contractants désignés:
**DE ES FR GB IT**

(30) Priorité: **06.04.2001 FR 0104743**

(43) Date de publication de la demande:
**16.10.2002 Bulletin 2002/42**

(73) Titulaire: **L'OREAL**
**75008 Paris (FR)**

(72) Inventeurs:
- **Chevalier, Véronique**
  **94440 Villecresnes (FR)**
- **Roger, Véronique**
  **92220 Bagneux (FR)**
- **Cassin, Guillaume**
  **91140 Villebon sur Yvette (FR)**

(74) Mandataire: **Catherine, Alain et al**
**Cabinet Harlé & Phélip**
**7, rue de Madrid**
**75008 Paris (FR)**

(56) Documents cités:
WO-A-00/73374          WO-A-97/35558
FR-A- 2 659 551        US-A- 3 819 825
US-A- 4 777 041        US-A- 5 702 714

- **PATENT ABSTRACTS OF JAPAN vol. 12, no. 50 (C-476), 16 février 1988 (1988-02-16) & JP 62 198608 A (SUMITOMO CEMENT CO), 2 septembre 1987 (1987-09-02)**

Remarques:
Le dossier contient des informations techniques présentées postérieurement au dépôt de la demande et ne figurant pas dans le présent fascicule.

**Description**

L'invention se rapporte à une composition cosmétique à effet anti-rides immédiat contenant, dans un milieu aqueux physiologiquement acceptable une quantité efficace d'au moins une charge minérale sous forme de particules colloïdales en dispersion dans un milieu aqueux.

La présente invention se rapporte également à l'utilisation dans et/ou pour la préparation d'une composition dermatologique, d'au moins une charge minérale comme agent tenseur pour tendre la peau et la lisser en vue d'atténuer immédiatement les rides et/ou ridules.

Par agent tenseur, on entend, au sens de la demande, un composé susceptible d'avoir un effet tenseur apparent, c'est-à-dire de lisser la peau et réduire, voire faire disparaître de façon immédiate les rides et les ridules.

Au cours du processus de vieillissement, il apparaît différents signes sur la peau, très caractéristiques de ce vieillissement, se traduisant notamment par une modification de la structure et des fonctions cutanées. Les principaux signes cliniques du vieillissement cutané sont notamment l'apparition de ridules et de rides profondes, en augmentation avec l'âge. On constate en particulier une désorganisation du "grain" de la peau, c'est-à-dire que le micro-relief est moins régulier et présente un caractère anisotrope.

Il est connu de traiter ces signes de vieillissement en utilisant des compositions cosmétiques ou dermatologiques contenant des actifs capables de lutter contre le vieillissement, tels que les α-hydroxy-acides, les β-hydroxy-acides et les rétinoïdes. Ces actifs agissent sur les rides en éliminant les cellules mortes de la peau et en accélérant le processus de renouvellement cellulaire.

Toutefois, l'effet visible de ces compositions ne se produit qu'au bout d'un certain temps d'application, pouvant aller de quelques jours à plusieurs semaines.

Il a donc été proposé, dans l'art antérieur, différentes compositions à effet tenseur permettant d'obtenir un lissage immédiat de la peau.

Ces compositions contiennent généralement, comme agent tenseur, des substances d'origine naturelle, telles que les dérivés végétaux, d'oeufs, de lait ou d'animaux. Ainsi, la demande internationale WO-98/29091 divulgue l'utilisation comme agent tenseur d'un système polymérique ayant des propriétés physico-chimiques particulières et comprenant au moins un polymère d'origine naturelle, tel qu'un extrait de protéine de soja ou des dérivés de chitine ou de kératine. En outre, la demande internationale WO 96/19180 décrit des compositions raffermissantes comprenant un agent filmogène contenant au moins un polysaccharide végétal et de la caséine hydrolysée comme actifs tenseurs. Toutefois, l'utilisation de substances d'origine naturelle est limitée par les risques d'encéphalopathie spongiforme bovine. De plus, outre qu'il n'est pas toujours reproductible, l'effet tenseur que procure ces substances n'est pas très important sur le plan qualitatif, et sur le plan quantitatif, il présente une faible rémanence.

D'autres compositions à effet tenseur de l'art antérieur utilisent des polymères synthétiques. On connaît ainsi la demande internationale WO-98/29022, une composition à effet tenseur comprenant une dispersion aqueuse d'un système polymérique contenant au moins un polymère d'origine synthétique, choisi parmi différents types de polyuréthannes, les polyurées, les polymères ou copolymères acryliques, les polymères d'acide isophtalique sulfoné et leurs mélanges. Le toucher cosmétique de ces compositions n'est cependant pas satisfaisant.

En outre, la demande de brevet européen EP-1038519-A1 décrit l'utilisation comme agent tenseur dans et/ou pour la fabrication d'une composition anti-rides, d'au moins un polymère siliconé greffé comprenant une portion polysiloxane et une position constituée d'une chaîne organique non siliconée, l'une des deux portions constituant la chaîne principale et l'autre étant greffé sur ladite chaîne principale. Toutefois, de tels polymères siliconés ne présentent pas d'effet anti-rides satisfaisant lorsqu'ils sont formulés en émulsion.

Par conséquent, il subsiste toujours le besoin de composés offrant un effet tenseur immédiat, suffisant et durable, sans risque pour le consommateur et pouvant être formulés dans des compositions comprenant des phases grasses.

La présente invention vise justement à la satisfaction d'un tel besoin.

Or, la demanderesse a découvert de manière surprenante que l'utilisation de charges minérales dans une composition cosmétique confère à cette composition, après application sur la peau, un excellent pouvoir lissant instantané de la couche superficielle de la peau lorsque ces charges sont sous forme de particules colloïdales en dispersion stable dans un milieu aqueux. Ces charges sous forme de particules colloïdales peuvent donc être utilisées en tant qu'agents tenseurs dans des compositions anti-rides à effet immédiat.

Par particules colloïdales, on entend, au sens de la demande, des particules ayant une taille de l'ordre de quelques dizaines de nanomètres.

L'homme de l'art connaît l'utilisation de charges minérales et plus particulièrement de la silice dans des compositions cosmétiques. Ainsi, par exemple, la silice est une matière largement utilisée dans l'industrie cosmétique. Elle apporte de la douceur, de la matité aux formulations. Elle est aussi utilisée en association avec du dioxyde de titane ou de l'oxyde de zinc pour former des particules complexes ayant des effets filtres solaires. Pour ces applications, la silice est très souvent sous forme pulvérulente, et une gamme importante en terme de taille de particules est disponible de 1 à 100 μm. Par contre, il est beaucoup moins habituel voire exceptionnel d'utiliser des particules colloïdales.

En ce qui concerne l'effet tenseur apporté par des charges minérales, la demande de brevet européen EP-1 008 340 décrit l'utilisation et la préparation à pH acide, de silicates mixtes en tant qu'agents tenseurs pour tendre la peau et la lisser en vue d'atténuer immédiatement. les rides et/ou ridules de la peau, les silicates mixtes étant sous forme de particules de taille moyenne allant de 15 à 1000 nm donnant des solutions colloïdales. Cependant, rien n'est dit dans cette demande concernant les charges autres que les silicates mixtes, et surtout, l'effet tenseur et le pouvoir lissant sont attribués à la nature chimique des silicates mixtes et non à la taille des particules.

Les brevets US 4,777,041 et US 3,819,825 décrivent tous deux des compositions cosmétiques pour le traitement des rides à base de silice.

Dans la composition de US 4,777,041, la silice, associée à une composition à base d'un polymère de polyuréthane, produit la gélification de cette composition. D'après l'enseignement de US 4,777,041, l'effet tenseur est attribué à l'action du gel ainsi obtenu, qui après application sur la peau ridée, sèche en remplissant les sillons des rides, de sorte que la peau apparaît alors lisse et uniforme.

Dans la composition de US 3,819,825, qui peut se présenter sous forme de gel ou d'aérosol, la silice est associée à une protéine entière constituée des composants d'une kératine. Lorsque la composition se présente sous forme de gel, l'application de la composition sur la peau conduit à la formation d'un film mince, tandis que l'on obtient une mousse lorsque la composition est sous forme d'aérosol. D'après l'enseignement US 3,819,825, l'effet tenseur est attribué au film ou à la mousse ainsi formés.

Toutefois, les compositions à effet anti-rides décrits dans US 4,777,041 et US 3.819,825 ne comprennent pas de phase grasse.

La présente invention a donc pour objet une composition cosmétique à effet anti-rides immédiat contenant, dans un milieu physiologiquement acceptable, une quantité efficace d'au moins une charge minérale caractérisée en ce que:

- la charge minérale est sous forme de particules colloïdales en dispersion dans un milieu aqueux, lesdites particules colloïdales étant choisies parmi les particules colloïdales de silice, d'oxyde de cérium, d'oxyde de zirconium, d'alumine, de carbonate de calcium, de sulfate de baryum, de sulfate de calcium, d'oxyde de zinc et de dioxyde de titane, les particules colloïdales de platine, et les particules colloïdales mixtes.
- au moins 70 % en nombre au moins ont un diamètre allant de 0,1 à 100 nm.
- lesdites particules colloïdales sont présentes dans la composition en une quantité de matière active allant de 0,5 à 15% en poids, et de préférence de 3 à 10% en poids par rapport au poids total de la composition,
- la composition est exempte de glycérine et de propylène glycol et se présente sous forme d'une émulsion eau-dans-huile (E/H) ou huile-dans-eau (H/E) ou eau-dans-huile-dans eau (E/H/E).

Par "milieu physiologiquement acceptable", on entend, au sens de la demande, un milieu non toxique et susceptible d'être appliqué sur la peau (y compris l'intérieur des paupières) ou les lèvres d'êtres humains.

Par milieu aqueux, on entend, au sens de la demande, aussi bien un milieu contenant seulement de l'eau qu'un milieu comprenant de l'eau et un solvant hydrosoluble tel qu'un alcool inférieur comportant 1 à 6 atomes de carbone ou un glycol.

Par quantité efficace, on entend, au sens de la demande, une quantité suffisante pour atteindre le but recherché, c'est-à-dire un effet tenseur et un pouvoir lissant instantané. Cette quantité dépend de la dispersion de charge minérale utilisée et des autres composés de la composition.

Par diamètre, on entend, au sens de la demande, le diamètre moyen en nombre.

La granulométrie est déterminée à l'aide d'un granulomètre Particle size analyser 90 Plus de Brookhaven Instrument Corporation.

Les particules colloïdales sont généralement présentes dans la composition en une quantité de matière active allant de 0,5 à 15% en poids, et de préférence de 3 à 10% en poids par rapport au poids total de la composition.

Les particules colloïdales de charge minérale utilisables selon l'invention sont de préférence non gélifiantes.

Par particules non gélifiantes, on entend, au sens de la demande, qu'à une concentration supérieure ou égale à 15% en poids par rapport au poids total de la composition, dans un milieu aqueux, alcoolique ou hydroalcoolique, la viscosité des solutions de particules colloïdales dans ledit milieu est inférieure à 0,05 Pa.s pour un taux de cisaillement de 10 s$^{-1}$, la mesure étant réalisée à 25°C à l'aide d'un rhéomètre Rhéostress RS150 de Haake en configuration cône-plan, le cône de mesure ayant un diamètre de 60 mm et un angle de 2°.

On utilisera de préférence dans la composition selon l'invention des silices colloïdales.

Par silices colloïdales, on entend, au sens de la demande, des particules colloïdales de silice en dispersion dans un milieu aqueux, hydroalcoolique, alcoolique.

Comme silices colloïdales utilisables dans la composition selon l'invention, on peut citer par exemple celles commercialisées par la société Catalysts et Chemicals sous les dénominations Cosmo S-40 et Cosmo S-50.

La phase huileuse de la composition selon l'invention comprend au moins une huile.

Comme huiles utilisables dans la composition de l'invention, on peut citer par exemple :

- les huiles hydrocarbonées d'origine animale, telles que le perhydrosqualène ;
- les huiles hydrocarbonées d'origine végétale, telles que les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique comme ceux vendus par la société Stearineries Dubois ou ceux vendus sous les dénominations Miglyol® 810, 812 et 818 par la société Dynamit Nobel, l'huile de jojoba, l'huile de beurre de karité ;
- les esters et les éthers de synthèse, notamment d'acides gras, comme les huiles de formules $R^1COOR^2$ et $R^1OR^2$ dans laquelle R1 représente le reste d'un acide gras comportant de 8 à 29 atomes de carbone, et $R^2$ représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle ; les esters hydroxylés comme l'isostéaryl lactate, l'octylhydroxystéarate, l'hydroxystéarate d'octyldodécyle, le diisostéaryl-malate, le citrate de triisocétyle, les heptanoates, octanoates, décanoates d'alcools gras ; les esters de polyol, comme le dioctanoate de propylène glycol, le diheptanoate de néopentylglycol et le diisononanoate de diéthylèneglycol ; et les esters du pentaérythritol comme le tétraisostéarate de pentaérythrityle ;
- les hydrocarbures linéaires ou ramifiés, d'origine minérale ou synthétique, tels que les huiles de paraffine, volatiles ou non, et leurs dérivés, la vaseline, les polydécènes, le polyisobutène hydrogéné tel que le Parléam® (de chez Nippon Gel Fats) ;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique ;
- les alcools gras alcoylés et notamment éthoxylés tels que l'oleth-12;
- les huiles fluorées partiellement hydrocarbonées et/ou siliconées comme celles décrites dans le document JP-A-2-295912. Comme huiles fluorées, on peut citer aussi le perfluorométhylcyclopentane et le perfluoro-1,3 diméthylcyclohexane, vendus sous les dénominations de "FLUTEC PC1®" et "FLUTEC PC3®" par la Société BNFL Fluorochemicals ; le perfluoro-1,2-diméthylcyclobutane ; les perfluoroalcanes tels que le dodécafluoropentane et le tétradécafluorohexane, vendus sous les dénominations de "PF 5050®" et "PF 5060®" par la Société 3M, ou encore le bromoperfluorooctyle vendu sous la dénomination "FORALKYL®" par la Société Atochem ; le nonafluoro-méthoxybutane vendu sous la dénomination "MSX 4518®" par la Société 3M et le nonafluoroéthoxyisobutane ; les dérivés de perfluoromorpholine, tels que la 4-trifluorométhyl perfluoromorpholine vendue sous la dénomination "PF 5052®" par la Société 3M ;
- les huiles de silicone comme les polyméthylsiloxanes (PDMS) volatiles ou non à chaîne siliconée linéaire ou cyclique, liquides ou pâteux à température ambiante, notamment les cyclopoly-diméthylsiloxanes (cyclométhicones) telles que la cyclohexa-siloxane ; les polydiméthylsiloxanes comportant des groupements alkyle, alcoxy ou phényle, pendant ou en bout de chaîne siliconée, groupements ayant de 2 à 24 atomes de carbone ; les silicones phénylées comme les phényltriméthicones, les phényldiméthicones, les phényltriméthylsiloxydiphényl-siloxanes, les diphényldiméthicones, les diphénylméthyldiphényl trisiloxanes, les 2-phényléthyltriméthyl-siloxysilicates, et les polyméthylphényl-siloxanes ;
- leurs mélanges.

On entend par « huile hydrocarbonée » dans la liste des huiles citées ci-dessus, toute huile comportant majoritairement des atomes de carbone et d'hydrogène, et éventuellement des groupements ester, éther, fluoré, acide carboxylique et/ou alcool.

La phase huileuse peut également comprendre d'autres corps gras, comme par exemple les acides gras comportant de 8 à 30 atomes de carbone, comme l'acide stéarique, l'acide laurique, l'acide palmitique et l'acide oléique ; les cires naturelles comme la lanoline, la cire d'abeille, la cire de Carnauba ou de Candellila, les cires de paraffine, de lignite ou les cires microcristallines, la cérésine ou l'ozokérite, les cires synthétiques comme les cires de polyéthylène, les cires de Fischer-Tropsch ; les gommes telles que les gommes de silicone (diméthiconol) ; les résines de silicone telles que la trifluorométhyl-$C_{1-4}$-alkyldimethicone et la trifluoropropyldimethicone ; et les élastomères de silicone comme les produits commercialisés sous les dénominations commerciales KSG® par la société Shin-Etsu, TREFIL®, BY29® et EPSX® par la société Dow Corning, ou sous la dénomination GRANSIL® par la société Grant Industries.

Ces corps gras peuvent être choisis de manière variée par l'homme du métier afin de préparer une composition ayant les propriétés, par exemple de consistance ou de texture, souhaitées.

Le pH de la composition va de préférence de 5 à 8, et mieux de 6 à 7. Il peut être ajusté par ajout d'acide, tel que par exemple l'acide chlorhydrique ou l'acide citrique.

En outre, les compositions utilisées selon l'invention peuvent être plus ou moins fluides et avoir l'aspect d'une crème blanche ou colorée, d'une pommade, d'un lait, d'une pâte, ou d'une mousse.

La proportion de la phase huileuse dans l'émulsion peut aller de 5 à 80 % en poids, et de préférence de 5 à 50 % en poids par rapport au poids total de la composition.

Les huiles, les émulsionnants et les co-émulsionnants utilisés dans la composition sous forme d'émulsion sont choisis parmi ceux classiquement utilisés dans le domaine cosmétique ou dermatologique. L'émulsionnant et le co-émulsionnant sont généralement présents dans la composition, en une proportion allant de 0,3 à 30 % en poids, et de préférence de 0,5 à 20 % en poids par rapport au poids total de la composition. L'émulsion peut, en outre, contenir des vésicules lipidiques. Les émulsions contiennent généralement au moins un émulsionnant choisi parmi les émulsionnants amphotères, anioniques, cationiques ou non ioniques, utilisés seuls ou en mélange. Les émulsionnants sont choisis de manière appropriée suivant l'émulsion à obtenir (E/H ou H/E).

Les émulsions peuvent également contenir des tensioactifs émulsionnants.

Comme tensioactifs émulsionnants utilisables pour la préparation des émulsions E/H, on peut citer par exemple les alkyl esters ou éthers de sorbitane, de glycérol ou de sucres ; les tensioactifs siliconés comme les dimethicone copolyols tels que le mélange de cyclomethicone et de dimethicone copolyol, vendu sous les dénominations DC 5225 C® ou DC 3225C® par la société Dow Corning, et les alkyl-dimethicone copolyols tels que le Laurylmethicone copolyol vendu sous la dénomination Dow Corning 5200 Formulation Aid® par la société Dow Corning, et le Cetyl dimethicone copolyol vendu sous la dénomination Abil EM 90® par la société Goldschmidt.

Pour les émulsions H/E, on peut citer par exemple comme émulsionnants, les émulsionnants non ioniques tels que les esters d'acides gras et de glycérol oxyalkylénés (plus particulièrement polyoxyéthylénés) ; les esters d'acides gras et de sorbitan oxyalkylénés ; les esters d'acides gras oxyalkylénés (oxyéthylénés et/ou oxypropylénés) ; les éthers d'alcools gras oxyalkylénés (oxyéthylénés et/ou oxypropylénés) ; les esters de sucres comme le stéarate de sucrose. On peut citer notamment le mélange de Dimyristyl tartrate/ cetearyl alcohol / $C_{12\text{-}15}$ Pareth-7 / PPG-25 Laureth-25 (nom CFTA) vendu sous la dénomination COSMACOL PSE® par la société CONDEA.

De façon connue, la composition cosmétique ou dermatologique de l'invention peut également contenir des adjuvants habituels dans le domaine cosmétique ou dermatologique, tels que les gélifiants hydrophiles ou lipophiles, les actifs hydrophiles ou lipophiles, les conservateurs, les antioxydants, les solvants, les parfums, les charges, les filtres solaires lipophiles ou hydrophiles, les bactéricides, les absorbeurs d'odeur, les matières colorantes, les sels, les polymères. Les quantités de ces différents adjuvants sont celles classiquement utilisées dans le domaine considéré, et par exemple de 0,01 à 30 % du poids total de la composition. Ces adjuvants, selon leur nature, peuvent être introduits dans la phase grasse, dans la phase aqueuse et/ou dans les sphérules lipidiques.

Comme charges qui peuvent être utilisées dans la composition de l'invention, on peut citer par exemple, outre les pigments, les fibres ; le talc ; les particules de polyamide et notamment celles vendues sous la dénomination ORGASOL® par la société Atochem ; les poudres de polyéthylène ; les microsphères à base de copolymères acryliques, telles que celles en copolymère diméthacrylate d'éthylène glycol/ methacrylate de lauryle vendues par la société Dow Corning sous la dénomination de POLYTRAP® ; les poudres expansées telles que les microsphères creuses et notamment, les microsphères commercialisées sous la dénomination EXPANCEL® par la société Kemanord Plast ou sous la dénomination MICROPEARL F 80 ED® par la société Matsumoto ; les poudres de matériaux organiques naturels tels que les amidons de maïs, de blé ou de riz, réticulés ou non, telles que les poudres d'amidon réticulé par l'anhydride octénylsuccinate, commercialisées sous la dénomination DRY-FLO® par la société National Starch ; les microbilles de résine de silicone telles que celles commercialisées sous la dénomination TOSPEARL® par la société Toshiba Silicone ; et leurs mélanges. Ces charges peuvent être présentes dans des quantités allant de 0 à 20 % en poids et de préférence de 1 à 10 % en poids par rapport au poids total de la composition.

Comme gélifiants hydrophiles, on peut citer par exemple les polymères carboxyvinyliques comme les produits commercialisés sous les dénominations CARBOPOL® (nom CTFA : carbomer) par la société Goodrich, les copolymères acryliques tels que les copolymères d'acrylates/alkylacrylates, les polyacrylamides et par exemple le mélange vendu sous la dénomination SEPIGEL®, les polymères et copolymères d'acide 2-acrylamido 2-méthylpropane sulfonique réticulés ou non, et neutralisés ou non, comme le produit le produit commercialisé par la société Hoechst sous la dénomination commerciale Hostacerin AMPS@ (nom CTFA : ammonium polyacryldimethyltauramide), les polysaccharides tels que les dérivés de cellulose et notamment l'hydroxyéthylcellulose, les gommes naturelles comme la gomme de xanthane, et les argiles.

Comme gélifiants lipophiles, on peut citer les argiles modifiées comme les bentones, les sels métalliques d'acides gras, les polymères et copolymères de méthyl vinyl éther et d'anhydride maléique comme les produits commercialisés sous les dénominations STABILEZE® par la société ISP (nom CTFA : PVM/MA Decadiene Crosspolymer), et les polyéthylènes.

Comme actifs, on peut citer en particulier tous les actifs connus pour leur activité sur le vieillissement de la peau, comme les agents kératolytiques ou prodesquamants, par exemple les α-hydroxy-acides comme les acides glycolique, lactique, malique, citrique, tartrique, mandélique, et leurs dérivés ; les β-hydroxy-acides comme l'acide salicylique et ses dérivés ; les α-céto-acides comme l'acide ascorbique ou vitamine C et ses dérivés ; les β-céto-acides ; les rétinoïdes comme le rétinol (vitamine A) et ses esters (palmitate), le rétinal, l'acide rétinoïque et ses dérivés.

Comme actifs, on peut citer aussi les vitamines, telles que par exemple les vitamines B3 ou PP (niacinamide), B5 (panthénol), E (tocophérol), K1, et les dérivés de ces vitamines et notamment leurs esters ; les agents anti-radicaux libres ; les filtres solaires ; les agents hydratants comme les polyols ; les céramides ; et les polymères tenseurs notamment organiques tels que les latex, les hydrolysats de protéines et les dérivés de chitine ; la DHEA et ses dérivés tels que l'alpha-hydroxy-DHEA ; le coenzyme Q10, les agents blanchissants et dépigmentants comme l'acide kojique, les dérivés de para-aminophénols, l'arbutine et leurs dérivés, et leurs mélanges.

Comme filtres solaires chimiques utilisables dans la composition de l'invention, la composition de l'invention peut comprendre tous les filtres UVA et UVB utilisables dans le domaine cosmétique.

Comme filtres UVB, on peut citer par exemple :

1. les dérivés de l'acide salicylique, en particulier le salicylate d'homomenthyle et le salicylate d'octyle ;

2. les dérivés de l'acide cinnamique, en particulier le p-méthoxycinnamate de 2-éthylhexyle, commercialisé par la société Givaudan sous la dénomination Parsol MCX® ;

3. les dérivés de (β'-β-diphénylacrylate liquides, en particulier l'α-cyano-α-β-diphénylacrylate de 2-éthylhexyle ou octocrylène, commercialisé par la société BASF sous la dénomination UVINUL N539® ;

4. les dérivés de l'acide p-aminobenzoïque ;

5. le 4-méthyl benzylidène camphre commercialisé par la société Merck sous la dénomination EUSOLEX 6300® ;

6. l'acide 2-phénylbenzimidazole 5-sulfonique commercialisé sous la dénomination EUSOLEX 232® par la société Merck ;

7. les dérivés de 1,3,5-triazine, en particulier :

- la 2,4,6-tris[p-(2'-éthylhexyl-1'-oxycarbonyl)anilino]-1,3,5-triazine commercialisé par la société BASF sous la dénomination UVINUL T150®, et
- le dioctyl butamido triazone commercialisé par la société Sigma 3V sous la dénomination UVASORB HEB®, et

8. les mélanges de ces filtres.

Comme filtres UVA, on peut citer par exemple :

1. les dérivés de dibenzoylméthane, en particulier le 4-(ter.-butyl) 4'-méthoxy dibenzoylméthane commercialisé par la société Givaudan sous la dénomination PARSOL 1789® ;

2. l'acide benzène 1,4 [di(3-méthylidènecampho-10-sulfonique)] éventuellement sous forme partiellement ou totalement neutralisée, commercialisé sous la dénomination MEXORYL SX® par la société Chimex ;

3. les dérivés de benzophénone, par exemple :

- la 2,4-dihydroxybenzophénone (benzophénone-1),
- la 2,2',4,4'-tétra-hydroxybenzophénone (benzophénone-2),
- la 2-hydroxy-4-méthoxy-benzophénone (benzophénone-3), commercialisé sous la dénomination UVINUL M40® par la société BASF,
- l'acide 2-hydroxy-4-méthoxy-benzophénone-5-sulfonique (benzophénone-4) ainsi que sa forme sulfonate (benzophénone-5), commercialisé par la société BASF sous la dénomination UVINUL MS40®,
- la 2,2'-dihydroxy-4,4'-diméthoxy-benzophénone (benzophénone-6),
- la 5-chloro-2-hydroxybenzophénone (benzophénone-7),
- la 2,2'-dihydroxy-4-méthoxy-benzophénone (benzophénone-8),
- le sel disodique du diacide 2,2'-dihydroxy-4,4'-diméthoxy-benzophénone-5,5'-disulfonique (benzophénone-9),
- la 2-hydroxy-4-méthoxy-4'-méthyl-benzophénone (benzophénone-10),
- la benzophénone-11,
- la 2-hydroxy-4-(octyloxy)benzophénone (benzophénone-12) ;

4. les dérivés silanes ou les polyorganosiloxanes à groupement benzophénone ;

5. les anthranilates, en particulier l'anthranilate de menthyle commercialisé par la société Haarman & Reiner sous la dénomination NEO HELIOPAN MA® ;

6. les composés comportant par molécule au moins deux groupes benzoazolyle ou au moins un groupe benzodiazolyle, en particulier l'acide 14-bis-benzimidazolyl-phenylèn-3,3',5,5'-tétrasulfonique ainsi que ses sels commercialisés par la société Haarman & Reimer ;

7. les dérivés siliciés de benzimidazolyl-benzazoles N-substitués ou de benzofuranyl-benzazoles, et en

- le 2-[1-[3-[1,3,3,3-tétraméthyl-1-[(triméthylsilyl)oxy]-disiloxanyl]propyl]-1H-benzimidazol-2-yl]-benzoxazole,
- le 2-[1-[3-[1,3,3,3-tétraméthyl-1-[(triméthylsilyl)oxy]-disiloxanyl]propyl]-1H-benzimidazol-2-yl]-benzothiazole,
- le 2-[1-(3-trimethylsilanyl-propyl)-1H-benzimidazol-2-yl]-benzoxazole,
- le 6-méthoxy-1,1'-bis-(3-triméthylsilanyl-propyl)-1H,1'H-[2,2'] bibenzimidazolyl-benzoxazole,
- le 2-[1-(3-trimethylsilanyl-propyl)-1H-benzimidazol-2-yl]-benzothiazole, qui sont décrits dans la demande de brevet EP-A-1 028 120 ;

8. les dérivés de triazine, et en particulier la 2,4-bis {[4-(2-éthyl-hexyloxy)-2-hydroxy]-phényl}-6-(4-méthoxy-phényl)-1,3,5-triazine commercialisé par la société Ciba Geigy sous la dénomination TINOSORB S, et le 2,2'-méthylènebis-[6-(2H benzotriazol-2-yl)4-(1,1,3,3-tétraméthylbutyl)phénol] commercialisé par la société Ciba Geigy sous la dénomination TINOSORB M® ; et

9. leurs mélanges.

On peut aussi utiliser un mélange de plusieurs de ces filtres, et un mélange de filtres UVB et de filtres UVA, et aussi des mélanges avec des filtres physiques.

Comme filtres physiques, on peut citer les oxydes de titane (dioxyde de titane amorphe ou cristallisé sous forme rutile et/ou anatase), de zinc, de fer, de zirconium, de cérium ou leurs mélanges. Ces oxydes métalliques peuvent être sous forme de particules ayant une taille micrométrique ou nanométrique (nano-pigments). Sous forme de nano-pigments, les tailles moyennes des particules vont par exemple de 5 à 100 nm. On utilise de préférence dans la composition de l'invention des nano-pigments.

On utilise de préférence comme filtre dans la composition de l'invention le p-méthoxycinnamate de 2-éthylhexyle commercialisé sous la dénomination PARSOL MCX® par la société Hoffmann-Laroche, l'acide benzène 1,4[di (3-méthylidènecampho-10-sulfonique)] commercialisé sous la dénomination MEXORYL SX® par la société Chimex, le 2-hydroxy-4-méthoxy-benzophénone commercialisé sous la dénomination UVINUL M40® par la société BASF, l'acide 2-phénylbenzimidazole-5 sulfonique commercialisé sous la dénomination EUSOLEX 232® par la société Merck, et leurs mélanges.

La quantité d'actifs dépend de l'utilisation finale souhaitée. Elle peut aller par exemple de 0,001 à 30 % en poids, de préférence de 0,05 à 20 % en poids et mieux de 0,5 à 15 % en poids par rapport au poids total de la composition.

L'invention permet le lissage de la peau et l'estompage des ridules et rides de la peau.

L'invention a donc aussi pour objet, un procédé de traitement cosmétique pour tendre la peau et la lisser en vue d'atténuer immédiatement les rides et/ou ridules, caractérisé en ce que l'on applique sur la peau une composition telle que définie ci-dessus.

L'invention a aussi pour objet un procédé de traitement cosmétique d'une peau ridée consistant à appliquer sur celle-ci une composition telle que définie ci-dessus, en une quantité efficace pour estomper immédiatement la ride ou la ridule par effet tenseur.

L'invention a encore pour objet l'utilisation d'une quantité efficace d'au moins une charge minérale, telle que définie précédemment comme agent tenseur dans et/ou pour la préparation d'une composition cosmétique exempte de glycérine et de propylène glycol et se présentant sous forme d'une émulsion eau-dans-huile (E/H) ou huile-dans-eau (H/E) ou eau-dans-huile-dans-eau (E/H/E).

L'invention sera illustrée à l'aide des exemples non limitatifs suivants. Les quantités sont indiquées en pourcentage en poids sauf mention contraire.

## <u>EXEMPLES</u>

**EXEMPLE 1** (en dehors du cadre de l'invention telle que revendiquée)

**Emulsion H/E**

On répare une émulsion H/E à partir des ingrédients indiqués dans le tableau 1.

## TABLEAU 1

|  | Noms CTFA | Quantité % en poids |
|---|---|---|
| Phase A | Tartrate de dimyristyle/ alcool cétéarylique/ $C_{12-15}$ pareth-7/PPG-25 Laureth-25 | 1,5 |
|  | Alcool stéarylique | 1 |
|  | Huiles | 10 |
|  | conservateur | 0,15 |
|  | méthoxy cinnamate d'éthylhexyle | 1 |
| Phase B | Glycérine | 5 |
|  | Conservateurs | 0,5 |
|  | Disodium EDTA | 0,05 |
|  | Ammonium polyacryl diméthyltauride (Hostacerin AMPS®) | 0,4 |
|  | Eau | qsp 100% |
| Phase C | gomme de xanthane | 0,2 |
| Phase D | Silice (COSMO S-40®) | 17,5 |

Mode opératoire:

- On chauffe la phase B à 75°C environ et on y incorpore l'ammonium poly acryl diméthylamide (Hostacerin AMPS®).
- On agite jusqu'à obtention d'un gel homogène.
- Puis on chauffe la phase A à environ 75°C.
- On réalise l'émulsion sous agitation en incorporant la phase A dans la phase B.
- On ajoute ensuite la phase C, toujours sous agitation.
- Puis, on incorpore à une température allant de 40 à 45°C la phase D et on maintient l'agitation jusqu'à refroidissement complet.

**EXEMPLE 2** (en dehors du cadre de l'invention telle que revendiquée)

**Sérum anti-vieillissement**

On prépare un sérum anti-vieillissement à partir des ingrédients indiqués dans le tableau 2.

## TABLEAU 2

| | Noms CTFA | Quantité % en poids |
|---|---|---|
| Phase A | Gomme de xanthane | 0,2% |
| | PVM/MA Decadiene Crosspolymer | 0,2% |
| | Conservateurs | Qs |
| | Eau | qsp 100% |
| Phase B | Triethanolamine | 0,2% |
| Phase C | Polyacrylamide, $C_{13}$-$C_{14}$ isoparaffine, et laureth-7 (SEPIGEL 305®) | 1% |
| Phase D | Silice (Cosmo S-40®) | 17,5% |

Mode opératoire

- On chauffe la phase A à environ 75°C et on disperse la gomme de xanthane et le polymère (PVM/MA Decadiene Crosspolymer) sous agitation.
- Puis on ajoute la phase B sous agitation.
- A 40°C, on ajoute la phase C.
- Puis on incorpore la phase D et on maintient l'agitation jusqu'à refroidissement complet.

Le caractère anti-rides immédiat est mis en évidence de la façon suivante :

- sur des modèles humains : on prend en photo les modèles avant l'application de tout produit, puis après avoir appliqué une émulsion contenant 7% (en matière active) de silice colloïdale et l'on compare les clichés. On constate une nette diminution de la visibilité des rides (particulièrement la patte d'oie et sous les yeux),
- par mesure à l'extensomètre; le pourcentage de rétraction de l'éprouvette de stratum corneum est de -1.3+/-0.5 pour la crème de l'exemple 1 et de - 2.2%/-0.4 pour le sérum de l'exemple

Evaluation de l'effet tenseur par mesure à l'extensomètre :

Le principe de la méthode consiste à mesurer la longueur d'une éprouvette de stratum corneum isolé à partir d'une peau humaine provenant d'une opération chirurgicale, avant et après traitement avec les compositions à tester.

Pour ce faire, l'éprouvette est placée entre les deux mâchoires de l'appareil, dont l'une est fixe et l'autre mobile, dans une atmosphère à 30°C et avec 40% d'humidité relative.

On exerce une traction sur l'éprouvette, et on enregistre la courbe de la force (en grammes) en fonction de la longueur (en millimètres), la longueur zéro correspondant au contact entre les deux mors de l'appareil.

On trace ensuite la tangente à la courbe dans sa région linéaire. L'intersection de cette tangente avec l'axe des abscisses correspond à la longueur apparente L0 de l'éprouvette à force nulle.

On détend l'éprouvette puis on applique sur le stratum corneum 2mg/cm$^2$ de la composition à tester. Après 15mn de séchage, les étapes ci-dessus sont à nouveau mises en oeuvre pour déterminer la longueur L1 de l'éprouvette après traitement.

Le pourcentage de rétraction est défini par ;

$$\% \text{ rétraction} = 100 \times (L1-L0)/L0$$

Pour caractériser un effet tenseur, ce pourcentage doit être négatif et l'effet tenseur est d'autant plus important que la valeur absolue du pourcentage de rétraction est élevé.

**EXEMPLE 3**

**Crèmes et sérums, anti-rides : influence de l'absence de glycérine**

On a préparé 2 crèmes anti-rides C1 et C2 contenant chacun 15% de silice colloïdale, à partir des ingrédients indiqués dans le tableau 3, la crème C1 (en dehors du cadre de l'invention telle que revendiquée) contenant 5 % en poids de glycérine par rapport au poids total de la composition, et le crème C2 en étant exempte.

On a également préparé 2 sérums anti-rides S1 et S2 contenant également chacun 15% de silice colloïdale, à partir des ingrédients indiqués dans le tableau 4, le sérum S1 (en dehors du cadre de l'invention telle que revendiquée) contenant 5% en poids de glycérine et le sérum S2 en étant exempt.

## TABLEAU 3

| | Noms CTFA | Crème C1 % en poids | Crème C2 % en poids |
|---|---|---|---|
| Phase A | Tartrate de dimyristyle/ alcool cétéarylique/ $C_{12\text{-}15}$ pareth-7/PPG-25 Laureth-25 | 1,5 | 1,5 |
| | Alcool stéarylique | 1 | 1 |
| | Huiles | 10 | 10 |
| | conservateur | 0,15 | 0,15 |
| | méthoxy cinnamate d'éthylhexyle | 1 | 1 |
| Phase B | Glycérine | 5 | 0 |
| | Conservateurs | 0,5 | 0,5 |
| | Disodium EDTA | 0,05 | 0,05 |
| | Ammonium polyacryl diméthyltauride (Hostacerin AMPS®) | 0,4 | 0,4 |
| | Eau | qsp 100% | qsp 100% |
| Phase C | gomme de xanthane | 0,2 | 0,8 |
| Phase D | Silice (COSMO S-40) | 15 | 15 |

## TABLEAU 4

| | Noms CTFA | Sérum S1 % en poids | Sérum S2 % en poids |
|---|---|---|---|
| Phase A | Gomme de xanthane | 0,2 | 0,2 |
| | Glycérine | 5 | 0 |
| | PVM/MA Decadiene Crosspolymer | 0,2 | 0,2 |
| | Conservateurs | qs | Qs |
| | Eau | qsp 100 | qsp 100 |
| Phase B | Triethanolamine | 0,2 | 0,2 |
| Phase C | Polyacrylamide, $C_{13}$-$C_{14}$ isoparaffine, et laureth-7 (SEPIGEL 305®) | 1 | 1 |
| Phase D | Silice (Cosmo S-40®) | 15 | 15 |

Le caractère anti-rides immédiat est mis en évidence, par mesure à l'extensomètre du pourcentage de rétraction d'une éprouvette de stratum cornéum isolé, de la même façon qu'à l'exemple 2.

Les résultats de ces mesures sont présentés dans le tableau 5.

TABLEAU 5

| Compositions | % de rétraction d'une éprouvette de stratum cornéum isolé |
|---|---|
| Crème C1 (avec glycérine) | $-1,15 \pm 0,21$ |
| Crème C2 (sans glycérine) | $-2,16 \pm 0,42$ |
| Sérum S1 (avec glycérine) | $-1,08 \pm 0,35$ |
| Sérum S2 (sans glycérine) | $-2,50 \pm 0,35$ |

Ces résultats montrent que le pouvoir tenseur des compositions cosmétiques contenant des particules colloïdales de charges minérale et de la glycérine est inférieur à celui de compositions identiques, mais ne contenant pas de glycérine.

**Revendications**

1. Composition cosmétique à effet anti-rides immédiat contenant, dans un milieu physiologiquement acceptable, une quantité efficace d'au moins une charge minérale à l'exclusion de tout silicate mixte, **caractérisée en ce que**

   - la charge minérale est sous forme de particules colloïdales en dispersion dans un milieu aqueux, lesdites particules colloïdales étant choisies parmi les particules colloïdales de silice, d'oxyde de cérium, d'oxyde de zirconium, d'alumine, de carbonate de calcium, de sulfate de baryum, de sulfate de calcium, d'oxyde de zinc et de dioxyde de titane, les particules colloïdales de platine, et les particules colloïdales mixtes,
   - au moins 70 % en nombre au moins ont un diamètre allant de 0,1 à 100 nm.
   - lesdites particules colloïdales sont présentes dans la composition en une quantité de matière active allant de 0,5 à 15% en poids, et de préférence de 3 à 10% en poids par rapport au poids total de la composition,

- la composition est exempte de glycérine et de propylène glycol et se présente sous forme d'une émulsion eau-dans-huile (E/H) ou huile-dans-eau (H/E) ou eau-dans-huile-dans eau (E/H/E).

2. Composition selon la revendication 1, **caractérisée en ce qu'**au moins 70% en nombre des particules ont un diamètre allant de 3 à 30 nm.

3. Composition selon la revendication 1 ou 2, **caractérisée en ce que** les particules colloïdales sont des silices colloïdales.

4. Composition selon l'une quelconque des revendications précédentes, **caractérisée en ce qu'**elle comprend une phase huileuse contenant au moins une huile choisie parmi les huiles hydrocarbonées d'origine végétale, les huiles hydrocarbonées d'origine animale, les huiles fluorées partiellement hydrocarbonées et/ou siliconées, les huiles de silicone, les hydrocarbures linéaires ou ramifiés, d'origine minérale ou synthétique, les alcools gras ayant de 8 à 26 atomes de carbone, les alcools gras alcoylés et notamment éthoxylés.

5. Composition selon la revendication 4, **caractérisée en ce que** la phase huileuse comprend également des corps gras choisis parmi les acides gras comportant de 8 à 30 atomes de carbone, les cires naturelles comme les cires d'abeille, de Carnauba ou de paraffine, les cires synthétiques, les gommes et les élastomères de silicone.

6. Composition selon l'une quelconque des revendications précédentes, **caractérisée en ce qu'**elle a un pH allant de 5 à 8 et de préférence de 6 à 7.

7. Composition selon l'une quelconque des revendications précédentes, **caractérisée en ce que** la phase huileuse représente de 5 à 80% en poids, et de préférence de 5 à 50% en poids du poids total de la composition.

8. Composition selon l'une quelconque des revendications précédentes, **caractérisée en ce qu'**elle comprend au moins un émulsionnant choisi parmi les émulsionnants amphotères, anioniques, cationiques ou non ioniques, utilisés seuls ou en mélange.

9. Composition selon l'une quelconque des revendications précédentes, **caractérisée en ce qu'**elle comprend un ou plusieurs adjuvants choisis parmi les gélifiants hydrophiles ou lipophiles, les actifs hydrophiles ou lipophiles, les conservateurs, les antioxydants, les solvants, les parfums, les charges, les filtres solaires lipophiles ou hydrophiles, les bactéricides, les absorbeurs d'odeur, les matières colorantes, les sels et les polymères, l'adjuvant étant présent à raison de 0,01 à 30% en poids par rapport au poids total de la composition.

10. Composition selon la revendication 9, **caractérisée en ce que** les gélifiants hydrophiles sont choisis parmi les polymères carboxyvinyliques, les copolymères acryliques, les polyacrylamides, les polymères et copolymères d'acide 2-acrylamido-2-méthylpropane sulfonique, les polysaccharides et les gommes naturelles.

11. Composition selon la revendication 9, **caractérisée en ce que** les gélifiants lipophiles sont choisis parmi les argiles modifiées, les sels métalliques d'acides gras, les polymères et copolymères de méthyl vinyl éther et d'anhydride maléique, et les polyéthylènes.

12. Composition selon la revendication 9, **caractérisée en ce que** les actifs sont choisis parmi les actifs agissant contre le vieillissement de la peau, les vitamines B3, B5, E, $K_1$ et leurs dérivés, et en particulier les esters, les agents anti-radicaux libres, les filtres solaires, les agents hydratants, les céramides, les polymères tenseurs notamment organiques, la DHEA et ses dérivés, la coenzyme Q10, les agents blanchissants et dépigmentants, et leurs mélanges.

13. Composition selon la revendication 9, **caractérisée en ce que** les actifs agissant contre le vieillissement de la peau sont choisis parmi les agents kératolytiques, les $\alpha$-hydroxy-acides, les $\alpha$-céto-acides, les $\beta$-céto-acides et les rétinoïdes.

14. Composition selon la revendication 12 ou 13, **caractérisée en ce que** les actifs sont présents à raison de 0,001 à 30% en poids, de préférence de 0,05 à 20% en poids, et mieux de 0,5 à 15% en poids par rapport au poids total de la composition.

15. Composition selon la revendication 9, **caractérisée en ce que** les charges sont choisies parmi les pigments, les fibres, le talc, les particules de polyamide, les microsphères à base de copolymères acryliques, les poudres expansées telles que les microsphères creuses, les poudres de matériaux organiques naturels, les microbilles de résine de silicone, les charges étant présentes dans la composition à raison de 0 à 20% en poids, et de préférence de 1 à 10% en poids par rapport au poids total de la composition.

16. Composition selon la revendication 12, **caractérisée en ce que** les filtres solaires sont choisis parmi les filtres UVA, les filtres UVB, les filtres physiques et leurs mélanges.

17. Procédé de traitement cosmétique pour tendre la peau et la lisser en vue d'atténuer immédiatement les rides et/ ou ridules, **caractérisé en ce que** l'on applique sur la peau une composition telle que définie selon l'une quelconque des revendications 1 à 16.

18. Utilisation d'au moins une charge minérale à l'exclusion de tout silicate mixte, sous forme de particules colloïdales en dispersion dans un milieu aqueux, dans une et/ou pour la préparation d'une composition cosmétique exempte de glycérine et de propylène glycol et se présentant sous forme d'une émulsion eau-dans-huile (E/H) ou huile-dans-eau (H/E) ou eau-dans-huile-dans-eau (E/H/E), comme agent tenseur pour tendre la peau et la lisser en vue d'atténuer immédiatement les rides et/ou ridules,
dans laquelle lesdites particules colloïdales sont choisies parmi les particules colloïdales de silice, d'oxyde de cérium, d'oxyde de zirconium, d'alumine, de carbonate de calcium, de sulfate de baryum, de sulfate de calcium, d'oxyde de zinc et de dioxyde de titane, les particules colloïdales de platine, et les particules colloïdales mixtes.

19. Utilisation selon la revendication 18, **caractérisée en ce que** la charge minérale se présente sous forme de particules colloïdales telles que 70 % en nombre au moins ont un diamètre allant de 3 à 30 nm, en dispersion dans un milieu aqueux, alcoolique ou hydroalcoolique.

20. Utilisation selon la revendication 18 ou 19, **caractérisée en ce que** les particules colloïdales sont des silices colloïdales.

**Patentansprüche**

1. Kosmetikzusammensetzung mit sofortiger Antifaltenwirkung, die in einem physiologisch annehmbaren Medium eine wirksame Menge wenigstens eines mineralischen Füllstoffs mit Ausnahme aller Mischsilikate enthält, **dadurch gekennzeichnet, dass**:

   - der mineralische Füllstoff in Form einer Dispersion von kolloidalen Teilchen in einem wässrigen Medium vorliegt, wobei die kolloidalen Teilchen ausgewählt sind aus kolloidalen Teilchen von Kieselsäure, Ceroxid, Zirconiumoxid, Aluminiumoxid, Calciumcarbonat, Bariumsulfat, Calciumsulfat, Zinkoxid und Titandioxid, kolloidalen Teilchen von Platin und gemischten kolloidalen Teilchen;

   - wenigstens 70 Zahlenprozent einen Durchmesser von 0,1 bis 100 nm haben;

   - die kolloidalen Teilchen in der Zusammensetzung in einer Wirkstoffmenge von 0,5 bis 15 Gew.-%, vorzugsweise 3 bis 10 Gew.-%, vorhanden sind, bezogen auf das Gesamtgewicht der Zusammensetzung;

   - die Zusammensetzung frei von Glycerin und Propylenglycol ist und in Form einer Wasser-in-Öl- (w/o) oder Öl-in-Wasser- (o/w) oder Wasser-in-Öl-in-Wasser-Emulsion (w/o/w) vorliegt.

2. Zusammensetzung gemäß Anspruch 1, **dadurch gekennzeichnet, dass** wenigstens 70 Zahlenprozent der Teilchen einen Durchmesser von 3 bis 30 nm haben.

3. Zusammensetzung gemäß Anspruch 1 oder 2, **dadurch gekennzeichnet, dass** es sich bei den kolloidalen Teilchen um kolloidale Kieselsäuren handelt.

4. Zusammensetzung gemäß einem der vorstehenden Ansprüche, **dadurch gekennzeichnet, dass** sie eine Ölphase umfasst, die wenigstens ein Öl enthält, das aus Kohlenwasserstoffölen pflanzlicher Herkunft, Kohlenwasserstoffölen tierischer Herkunft, fluorierten, partiellen Kohlenwasserstoffund/oder Silikonölen, Silikonölen, linearen oder verzweigten Kohlenwasserstoffen mineralischer oder synthetischer Herkunft, Fettalkoholen mit 8 bis 26 Kohlenstoffatomen, alkoxylierten und insbesondere ethoxylierten Fettalkoholen ausgewählt ist.

5. Zusammensetzung gemäß Anspruch 4, **dadurch gekennzeichnet, dass** die Ölphase auch Fettkörper umfasst, die aus Fettsäuren mit 8 bis 30 Kohlenstoffatomen, natürlichen Wachsen, wie Bienen-, Carnauba- oder Paraffinwachsen, synthetischen Wachsen, Gummen und Silikonelastomeren ausgewählt sind.

6. Zusammensetzung gemäß einem der vorstehenden Ansprüche, **dadurch gekennzeichnet, dass** sie einen pH-Wert von 5 bis 8 und vorzugsweise 6 bis 7 hat.

7. Zusammensetzung gemäß einem der vorstehenden Ansprüche, **dadurch gekennzeichnet, dass** die Ölphase 5 bis 80 Gew.-%, vorzugsweise 5 bis 50 Gew.-%, des Gesamtgewichts der Zusammensetzung ausmacht.

8. Zusammensetzung gemäß einem der vorstehenden Ansprüche, **dadurch gekennzeichnet, dass** sie wenigstens einen Emulgator umfasst, der aus amphoteren, anionischen, kationischen oder nichtionischen Emulgatoren ausgewählt ist, die allein oder im Gemisch verwendet werden.

9. Zusammensetzung gemäß einem der vorstehenden Ansprüche, **dadurch gekennzeichnet, dass** sie einen oder mehrere Hilfsstoffe umfasst, die aus hydrophilen oder lipophilen Geliermitteln, hydrophilen oder lipophilen Aktivstoffen, Konservierungsmitteln, Antioxidantien, Lösungsmitteln, Duftstoffen, Füllstoffen, hydrophilen oder lipophilen Sonnenfiltern, Bakteriziden, Geruchsabsorbern, Farbstoffen, Salzen und Polymeren ausgewählt sind, wobei der Hilfsstoff in einer Menge von 0,01 bis 30 Gew.-% vorhanden ist, bezogen auf das Gesamtgewicht der Zusammensetzung.

10. Zusammensetzung gemäß Anspruch 9, **dadurch gekennzeichnet, dass** die hydrophilen Geliermittel aus Carboxyvinylpolymeren, Acrylcopolymeren, Polyacrylamiden, Polymeren und Copolymeren von 2-Acrylamido-2-methylpropansulfonsäure, Polysacchariden und Naturgummen ausgewählt sind.

11. Zusammensetzung gemäß Anspruch 9, **dadurch gekennzeichnet, dass** die lipophilen Geliermittel aus modifizierten Tonen, Metallsalzen von Fettsäuren, Polymeren und Copolymeren von Methylvinylether und Maleinsäureanhydrid und Polyethylenen ausgewählt sind.

12. Zusammensetzung gemäß Anspruch 9, **dadurch gekennzeichnet, dass** die Aktivstoffe aus Aktivstoffen, die gegen die Hautalterung wirken, den Vitaminen B3, B5, E, $K_1$ und ihren Derivaten und insbesondere Estern, Radikalfängern, Sonnenfiltern, Hydratisierungsmitteln, Ceramiden, straffenden Polymeren, insbesondere organischen straffenden Polymeren, DHEA und seinen Derivaten, Coenzym Q10, Bleich- und Depigmentierungsmitteln und ihren Gemischen ausgewählt sind.

13. Zusammensetzung gemäß Anspruch 9, **dadurch gekennzeichnet, dass** die Aktivstoffe, die gegen die Hautalterung wirken, aus keratolytischen Mitteln, $\alpha$-Hydroxysäuren, $\alpha$-Ketosäuren, $\beta$-Ketosäuren und Retinoiden ausgewählt sind.

14. Zusammensetzung gemäß Anspruch 12 oder 13, **dadurch gekennzeichnet, dass** die Aktivstoffe in einer Menge von 0,001 bis 30 Gew.-%, vorzugsweise 0,05 bis 20 Gew.-% und besonders bevorzugt 0,5 bis 15 Gew.-% vorhanden sind, bezogen auf das Gesamtgewicht der Zusammensetzung.

15. Zusammensetzung gemäß Anspruch 9, **dadurch gekennzeichnet, dass** die Füllstoffe aus Pigmenten, Fasern, Talk, Polyamidteilchen, Mikrosphären auf der Basis von Acrylcopolymeren, expandierten Pulvern, wie Mikrohohlkugeln, Pulvern aus natürlichen organischen Materialien, Silikonharzmikrokugeln ausgewählt sind, wobei die Füllstoffe in der Zusammensetzung in einer Menge von 0 bis 20 Gew.-%, vorzugsweise 1 bis 10 Gew.-%, vorhanden sind, bezogen auf das Gesamtgewicht der Zusammensetzung.

16. Zusammensetzung gemäß Anspruch 12, **dadurch gekennzeichnet, dass** die Sonnenfilter aus UVA-Filtern, UVB-Filtern, physikalischen Filtern und ihren Gemischen ausgewählt sind.

17. Verfahren zur kosmetischen Behandlung zur Straffung und Glättung der Haut, um Falten und/oder Fältchen sofort zu reduzieren, **dadurch gekennzeichnet, dass** man eine Zusammensetzung gemäß einem der Ansprüche 1 bis 16 auf die Haut aufträgt.

18. Verwendung wenigstens eines mineralischen Füllstoffs mit Ausnahme aller Mischsilikate in Form einer Dispersion von kolloidalen Teilchen in einem wässrigen Medium bei der und/oder zur Herstellung einer Kosmetikzusammensetzung, die frei von Glycerin und Propylenglycol ist und in Form einer Wasser-in-Öl- (w/o) oder Öl-in-Wasser- (o/w) oder Wasser-in-Öl-in-Wasser-Emulsion (w/o/w) vorliegt, als Straffungsmittel, um die Haut zu straffen und zu glätten, um Falten und/oder Fältchen sofort zu reduzieren, wobei die kolloidalen Teilchen ausgewählt sind aus kolloidalen Teilchen von Kieselsäure, Ceroxid, Zirconiumoxid, Aluminiumoxid, Calciumcarbonat, Bariumsulfat, Calciumsulfat, Zinkoxid und Titandioxid, kolloidalen Teilchen von Platin und gemischten kolloidalen Teilchen.

19. Verwendung gemäß Anspruch 18, **dadurch gekennzeichnet, dass** der mineralische Füllstoff in Form von kolloidalen Teilchen, von denen wenigstens 70 Zahlenprozent einen Durchmesser von 3 bis 30 nm haben, in Dispersion in einem wässrigen, alkoholischen oder wässrig-alkoholischen Medium vorliegt.

20. Verwendung gemäß Anspruch 18 oder 19, **dadurch gekennzeichnet, dass** es sich bei den kolloidalen Teilchen um kolloidale Kieselsäuren handelt.

## Claims

1. A cosmetic composition having an immediate anti-wrinkle effect, containing, in a physiologically acceptable medium, an efficient amount of at least one mineral filler, with the exclusion of any mixed silicate, **characterized in that** :

   - the mineral filler is in the form of colloid particles dispersed in an aqueous medium, said colloid particles are selected from silica, cerium oxide, zirconium oxide, alumina, calcium carbonate, barium sulfate, calcium sulfate, zinc oxide and titanium dioxide colloid particles, platinum colloid particles and mixed colloid particles,
   - at least 70 % of the particles have a diameter in the range from 0.1 to 100nm;
   - said colloid particles are present in the composition in an active material amount ranging from 0.5 to 15% wt, and preferably from 3 to 10 % wt, based on the total weight of the composition ;
   - the composition is free from glycerin and propylene glycol and is in the form of a water-in-oil emulsion (W/O) or an oil-in-water emulsion (O/W) or a water-in-oil-in-water (W/O/W) emulsion,

2. A composition according to claim 1, **characterized in that** at least 70% of the particles have a diameter in the range from 3 to 30 nm.

3. A composition according to claim 1 or 2, **characterized in that** the colloid particles are colloidal silicas.

4. A composition according to anyone of the preceding claims, **characterized in that** it comprises an oil phase containing at least one oil selected among hydrocarbon oils from plant origin, hydrocarbon oils from animal origin, partially hydrocarbon and/or silicone fluorinated oils, silicone oils, linear or branched hydrocarbons, from mineral or synthetic origin, fatty alcohols having 8 to 26 carbon atoms, alkylated and more particularly ethoxylated fatty alcohols.

5. A composition according to claim 4, **characterized in that** the oil phase also comprises fatty bodies selected amongst fatty acids having 8 to 30 carbon atoms, natural waxes such as beewax, Carnauba or paraffin waxes, synthetic waxes, gums and silicone elastomers.

6. A composition according to anyone of the preceding claims, **characterized in that** it has a pH ranging from 5 to 8, preferably from 6 to 7.

7. A composition according to anyone of the preceding claims, **characterized in that** the oil phase represents from 5 to 80% wt, and preferably from 5 to 50% wt, based on the total weight of the composition.

8. A composition according to anyone of the preceding claims, **characterized in that** it comprises at least one emulsifier selected amongst amphoteric, anionic, cationic or non ionic emulsifiers, used alone or in blends.

9. A composition according to anyone of the preceding claims, **characterized in that** it contains one or more adjuvants, selected among hydrophilic or lipophilic gelling agents, hydrophilic or lipophilic actives, preservatives, antioxidants, solvents, perfumes, fillers, lipophilic or hydrophilic sun filters, bactericides, odour absorbents, dyes, salts, polymers, the adjuvants being present in the range from 0.01 to 30% wt based on the total weight of the composition.

10. A composition according to claim 9, **characterized in that** the hydrophilic gelling agents are selected amongst carboxyvinyl polymers, acrylic copolymers, polyacrylamides, 2-acrylamido-2-methylpropane sulfonic acid polymers and copolymers, polysaccharides and natural gums.

11. A composition according to claim 9, **characterized in that** the lipophilic gelling agents are selected amongst modified clays, fatty acid metallic salts, vinyl methyl ether and maleic anhydrid polymers and copolymers, and polyethylenes.

12. A composition according to claim 9, **characterized in that** the actives are selected amongst actives acting against skin ageing, vitamins B3, B5, E, $K_1$, and their derivatives and in particular the esters, anti-free radical agents, sun filters, hydrating agents, ceramides, tensing polymers, including organics, DHEA, and the derivatives thereof, Q10 coenzyme, bleaching and depigmenting agents, and the blends thereof.

13. A composition according to claim 9, **characterized in that** the actives acting against skin ageing are selected amongst keratolytic agents, $\alpha$-hydroxy-acids, $\alpha$-ceto-acids, $\beta$-ceto-acids and retinoids.

14. A composition according to claim 12 or 13, **characterized in that** the actives are present in a range from 0.001 to 30% wt, preferably from 0.05 to 20 % wt, and more preferably from 0.5 to 15% wt, based on the total weight of the composition.

15. A composition according to claim 9, **characterized in that** the fillers are selected amongst pigments, fibres, talcum, polyamide particles, acrylic copolymer based microspheres, expanded powders such as hollow microspheres, powders from natural organic materials, silicone resin microballs, the fillers being present in the composition in amounts ranging from 0 to 20% wt, and preferably from 1 to 10% wt, based on the total weight of the composition.

16. A composition according to claim 12, **characterized in that** the sun filters are selected amongst the UVA filters, the UVB filters, the physical filters and the blends thereof.

17. A cosmetic treatment method for tensing and smoothing skin for immediately attenuating wrinkles and/or fine wrinkles, **characterized in that** a composition such as defined according to anyone of claims 1 to 16 is applied onto the skin.

18. Use of at least one mineral filler, with the exclusion of any mixed silicate, in the form of colloid particles dispersed in an aqueous medium, in and/or for preparing a cosmetic composition free from glycerin and propylene glycol and which is in the form of a water-in-oil emulsion (W/O) or an oil-in-water emulsion (O/W) or a water-in-oil-in-water (W/O/W) emulsion as a tensing agent for tensing and smoothing skin in order to immediately attenuate wrinkles and/or fine wrinkles, wherein said colloid particles are selected from silica, cerium oxide, zirconium oxide, alumina, calcium carbonate, barium sulfate, calcium sulfate, zinc oxide and titanium dioxide colloid particles, platinium colloid particles and mixed colloid particles.

19. Use according to claim 18, **characterized in that** the mineral filler is in the form of colloid particles such as 70% of them at least have a diameter ranging from 3 to 30 nm in a dispersion in an aqueous, alcoholic or hydroalcoholic medium.

20. Use according to claim 18 or 19, **characterized in that** the colloid particles are colloidal silicas.